# EUROPÄISCHE PATENTSCHRIFT

(11) **EP 2 649 981 B1**
(45) Veröffentlichungstag und Bekanntmachung des Hinweises auf die Patenterteilung: **21.11.2018**
(21) Anmeldenummer: 12163823.3
(22) Anmeldetag: 11.04.2012
(51) Int. Cl.: A61K 6/00

(54) **Polymerisierbare Zusammensetzungen mit hoher Polymerisationstiefe**
Polymerisable compounds with high polymerisation depth
Compositions polymérisables avec profondeur de polymérisation élevée

(43) Veröffentlichungstag der Anmeldung: 16.10.2013
(73) Patentinhaber: Ivoclar Vivadent AG, 9494 Schaan (LI)
(72) Erfinder: Burtscher, Dr. Peter, 6830 Rankweil (AT); Moszner, Prof. Dr. Norbert, 9495 Triesen (LI); Rheinberger, Dr. Volker, 9490 Vaduz (LI)
(74) Vertreter: Uexküll & Stolberg

(56) Entgegenhaltungen:
- EP-A1- 2 233 449
- US-A1- 2011 054 066
- US-A1- 2011 118 380
- US-A1- 2012 010 322

## Beschreibung

Die vorliegende Erfindung betrifft polymerisierbare Zusammensetzungen, die eine Photoinitiatormischung aufweisen, welche neben einem α-Diketon und einem Beschleuniger mindestens eine Diacylgermanium-Verbindung enthält. Die Zusammensetzungen eignen sich besonders zur Herstellung von dentalen Adhäsiven, Zementen und Füllungskompositen.

Lichthärtende Kompositrestaurationen haben heutzutage in der zahnärztlichen Füllungstherapie eine sehr dominante Stellung erreicht (vgl. N. Ilie, R. Hickel, Clin. Oral Invest. 13 (2009) 427-438). Dabei erfolgt die Aushärtung der Komposite meist durch Bestrahlung mit Blaulicht im Wellenlängenbereich von ca. 400-500 nm unter Verwendung von verschieden Strahlungsquellen, vor allem von Halogenlampen oder LEDs (light emitting diodes), aber auch von Argonionen-Lasern oder Plasmabogengeräten (vg. F. Rueggeberg, Dental Materials 27 (2011) 39-52). Die Füllungskomposite basieren weitgehend auf gefüllten Dimethacrylatharzen, die eine Mischung aus Campherchinon (CQ) und einem Aminbeschleuniger als Photoinitiatorssystem enthalten können (N. Moszner, R. Liska: Photoinitiators for direct adhesive restorative materials, in Basics and Application of Photopolymerization Reactions, Vol. 1, Eds.: J. P. Fourassier, X. Allonas, Research Signpost, Kerala 2010, 91-112).

Für die klinische Performance der Füllungskomposite ist eine gute Polymerisationstiefe von zentraler Bedeutung. Nach der ISO 4049 - 2009 ("Dentistry - Polymer-based restorative materials") wird die Polymerisationstiefe (depth of cure) so bestimmt, dass ein zylindrischer Kompositprobekörper in einer Stahlform die empfohlene Zeit bestrahlt wird. Dann wird der Probekörper aus der Form genommen und das nichtpolymerisierte Komposit mit einem Kunststoffspatel entfernt. Die Höhe des verbleibenden Zylinders, dividiert durch 2, wird als Polymerisationstiefe bezeichnet und ist quasi ein Maß dafür, wie wirksam das Komposit durch das eingestrahlte Licht ausgehärtet werden kann.

Die Polymerisationstiefe ist sowohl von Prozessparametern als auch von den Materialeigenschaften abhängig. So besteht z.B. zwischen Polymerisationstiefe und der Intensität des eingestrahlten Lichtes bzw. der Belichtungszeit ein logarithmischer Zusammenhang (vgl. J. H. Lee, R. K. Prud'homme, I. A. Aksay, J. Mater. Res. 16 (2001) 3536-3544). Dabei sollte das Emissionsspektrum der Strahlungsquelle mit dem Absorptionsspektrum des Photoinitiators gut übereinstimmen. Weiterhin korreliert die Polymerisationstiefe mit der Transluzens des Komposites, die wiederum u.a durch den Brechungsindex der Harzmatrix und der Füllstoffe, durch die Grösse der Füllstoffpartikel sowie die Art und Konzentration zugesetzter Farbmittel beeinflusst wird (E. Mahn, Zahnmedizin 2011, 50-59). Außerdem wird die Polymerisationstiefe durch die Art und Konzentration des Photoinitiatorsystems beeinflusst, wobei sich monomolekulare Photoinitiatoren einfacher kontrollieren lassen als bimolekulare Photoinitiatorsysteme. Beispiele für monomolekulare Photoinitiatoren sind die kommerziell erhältlichen Verbindungen 2,4,6-Trimethylbenzoyldiphenylphosphinoxid (λₘₐₓ = 365, nm) und Bis (2, 4, 6-trimethylbenzoyl)phenylphosphinoxid (λₘₐₓ = 397 nm). Diese Verbindungen können allein eingesetzt werden oder mit anderen Photoinitiatoren kombiniert werden, um eine schnellere Polymerisation der Harzmatrix oder eine Aushärtung in zwei Schritten zu erreichen (vgl. EP 184 095 B1, DE 38 01 511 A1). Die Verwendung von Acylgermanen, die als monomolekulare Photoinitiatoren im sichtbaren Bereich eingesetzt werden können und sich durch eine hohe Durchhärtungstiefe auszeichnen, ist in der EP 1 905 413 A1 offenbart.

Die US 2011/0118380 A1 offenbart polymerisierbare Dentalwerkstoffe, die mindestens eine radikalisch polymerisierbare Verbindung und einen Photoinitiator auf Basis von Campherchinon/Amin, Bisacylphosphinoxid, einer Diacyldialkylgermanium-Verbindung oder einer Mischung davon enthalten.

Die US 2011/0054066 A1 offenbart polymerisierbare Dentalwerkstoffe, die mindestens einen radikalisch polymerisierbaren macrocyclischen Polyether und eine Mischung verschiedener Photoinitiatoren enthalten, beispielsweise eine Mischung von Dibenzoyldiethylgermanium, Campherchinon und 4-Dimethylaminobenzoesäureethylester oder eine Mischung von 0,2 Gew.-% Campherchinon, 0,5 Gew-% 4-Dimethylaminobenzoesäureethylester und 1,0 Gew.-% 2,4,6-Trimethylbenzoyldiphenylphosphinoxid.

Die EP 2 233 449 A1 offenbart einen wachshaltigen Schlicker, der mindestens ein radikalisch polymerisierbares Monomer und als Polymerisationsinitiator beispielsweise eine Mischung von Dibenzoyldiethylgermanium, Campherchinon und 4-Dimethylaminobenzoesäureethylester enthält.

Die US 2021/0010322 A1 offenbart polymerisierbare Dentalwerkstoffe, die eine Mischung verschiedener Photoinitiatoren enthalten, beispielsweise eine Mischung von Dibenzoyldiethylgermanium, Campherchinon und 4-Dimethylaminobenzoesäureethylester.

Der Erfindung liegt die Aufgabe zugrunde, Polymerisationsinitiatorsysteme zur Verfügung zu stellen, die mit sichtbarem Licht aktiviert werden können und die eine verbesserte Durchhärtungstiefe des zu härtenden Materials ergeben. Insbesondere sollen polymerisierbare Zusammensetzungen bereitgestellt werden, die sich als Komposite für dentale Anwendungen eignen und sich durch eine verbesserte Polymerisationstiefe, ein sehr gutes Bleichverhalten, eine relativ schnelle Aushärtung und sehr gute mechanische Eigenschaften des ausgehärteten Materials auch in tieferen Schichten auszeichnen.

Erfindungsgemäß wird diese Aufgabe durch Zusammensetzungen mit mindestens einem polymerisierbaren Bindemittel gelöst, wobei die Zusammensetzungen eine Photoinitiatormischung aufweisen, die, bezogen auf das polymerisierbare Bindemittel,
(a) 0,2 bis 0,5 Gew.-% mindestens einer Diacylgermanium-Verbindung der Formel (I),
(b) 0,2 bis 0,5 Gew.-% mindestens eines α-Diketons und
(c) 0,4 bis 0,8 Gew.-% mindestens eines Beschleunigers, insbesondere eines Aminbeschleunigers, und
(d) 0,1 bis 0,6 Gew.-% mindestens eines Acyl- oder Bisacylphosphinoxids
enthält, 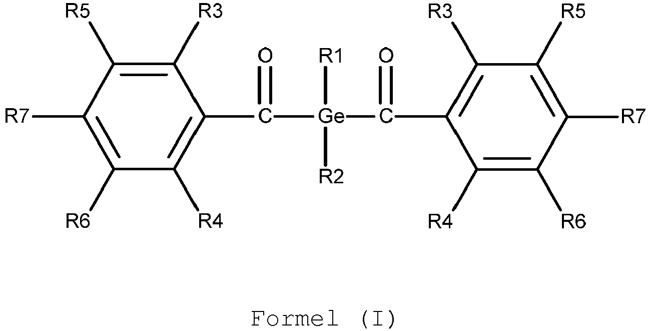 in der
- R¹, R²: unabhängig voneinander ein C₁₋₄-Alkyl-, oder C₂₋₄-Alkenyl-Rest sind, der durch eine oder mehrere polymerisationsfähige Gruppen substituiert sein kann;
- R³, R⁴: unabhängig voneinander jeweils H, Halogen, ein verzweigter oder linearer C₁₋₄-Alkyl- oder -O-C₁₋₄-Alkylrest sind;
- R⁵, R⁶: unabhängig voneinander jeweils H, Halogen, ein linearer C₁₋₂₀-Alkyl-, -Alkenyl-, -Alkoxy- oder -Alkenoxy-Rest sind, der durch ein oder mehrere -O-, -S- oder -NR⁸-Reste unterbrochen und durch ein oder mehrere polymerisationsfähige Gruppen substituiert sein kann;
- R⁷: unabhängig voneinander jeweils H, Halogen, ein linearer C₁₋₂₀-Alkyl-, -Alkenyl-, -Alkoxy- oder -Alkenoxy-Rest ist, der durch ein oder mehrere-O-, -S- oder -NR⁸-Reste unterbrochen und durch ein oder mehrere polymerisationsfähige Gruppen substituiert sein kann; und
- R⁸: unabhängig jeweils H, ein C₁₋₂₀-Alkyl-, -Alkenyl-, -Alkoxy- oder -Alkenoxy-Rest ist.

Es wurde überraschenderweise gefunden, dass die Verwendung einer erfindungsgemäßen Kombination von Photoinitiatoren bei der Bestrahlung von polymerisierbaren Bindemitteln mit Licht, vorzugsweise im sichtbaren Bereich, insbesondere mit einer Wellenlänge von 380 bis 500 nm, im Vergleich zu herkömmlichen Photoinitiatoren zu einer verbesserten Polymerisationstiefe und zu sehr guten mechanischen Eigenschaften auch in tieferen Schichten des auszuhärtenden Materials führt. Der Begriff "Photoinitiator" soll sich hier auf Substanzen und Substanzgemische beziehen, die bei Bestrahlung mit Licht Radikale bilden und somit eine Polymerisation des polymerisierbaren Bindemittels auslösen können oder eine derartige Polymerisation beschleunigen können, und soll insbesondere monomolekulare und bimolekulare Photoinitiatoren einschließen.

Die Photoinitiatormischung der Zusammensetzung enthält neben einem oder mehreren α-Diketonen und einem oder mehreren Beschleunigern mindestens eine spezielle Diacylgermanium-Verbindung der Formel (I). Durch die Formel (I) und die übrigen hierin gezeigten Formeln werden sämtliche stereoisomere Formen sowie Gemische verschiedener stereoisomerer Formen, wie z.B. Racemate, erfasst. Die Formeln erfassen nur solche Verbindungen, die mit der chemischen Valenzlehre vereinbar sind. Der Hinweis, dass ein Rest durch ein Heteroatom wie O unterbrochen sein kann, ist so zu verstehen, dass die O-Atome in die Kohlenstoffkette des Restes eingeschoben werden, d.h. beidseitig von Kohlenstoffatomen begrenzt werden. Die Anzahl der O-Atome ist daher um mindestens 1 kleiner als die Zahl der Kohlenstoffatome, und die O-Atome können nicht endständig sein. Erfindungsgemäß sind Reste, die nicht durch O-Atome unterbrochen sind, bevorzugt.

Halogen (abgekürzt Hal) steht vorzugsweise für F, Cl, Br oder I, insbesondere für F, Cl, ganz besonders bevorzugt für Cl. Bevorzugte polymerisationsfähige Gruppen, die bei den obigen Resten als Substituenten vorhanden sein können, sind Vinyl, Styryl, (Meth)acrylat, (Meth)acrylamid und/oder N-Alkylacrylamid, besonders bevorzugt (Meth)acrylat, (Meth)acrylamid und/oder N-Alkylacrylamid.

Erfindungsgemäß sind solche Zusammensetzungen bevorzugt, die als Bestandteil (a) eine Diacylgermaniumverbindung der Formel (II) enthalten: 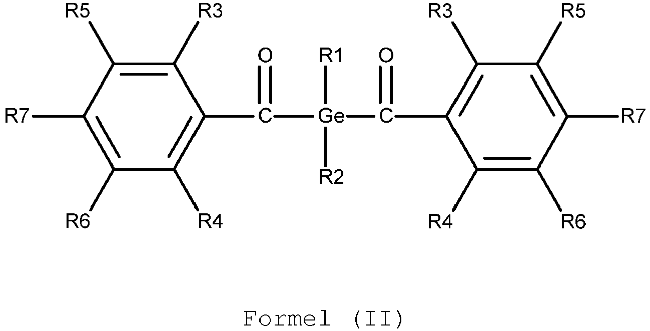 in der
- R¹, R²: unabhängig voneinander ein linearer C₁₋₄-Alkyl-Rest sind;
- R³, R⁴: jeweils H sind;
- R⁵, R⁶: jeweils H sind; und
- R⁷: unabhängig voneinander jeweils H, ein linearer C₁₋₆-Alkyl- oder -Alkoxy-Rest, vorzugsweise unabhängig voneinander jeweils H oder ein C₁₋₆-Alkoxy-Rest ist.

Besonders bevorzugt ist die Diacylgermaniumverbindung (a) ausgewählt aus der Gruppe bestehend aus Bis(4-methoxybenzoyl)diethylgermanium, Dibenzoyldiethylgermanium und Mischungen davon. Ganz besonders bevorzugt enthält die Zusammensetzung Bis(4-methoxybenzoyl)diethylgermanium als Diacylgermanium-Verbindung (a).

Als α-Diketone lassen sich vorzugsweise 9,10-Phenanthrenchinon, 1-Phenylpropan-1,2-dion, Diacetyl, 4,4'-Dichlorbenzil, Campherchinon oder deren Derivate einsetzen. Insbesondere enthält die Photoinitiatormischung ein α-Diketon, das ausgewählt ist aus der Gruppe bestehend aus Campherchinon, 1-Phenylpropan-1,2-dion und Mischungen davon.

Als Beschleuniger eignen sich Aminbeschleuniger und/oder aminfreie Beschleuniger. Es ist besonders bevorzugt, dass die erfindungsgemäße Zusammensetzung als Beschleuniger (c) mindestens einen Aminbeschleuniger enthält.

Als Aminbeschleuniger können tertiäre Amine verwendet werden, wie z.B. tertiäre aromatische Amine, wie N,N-Dialkyl-aniline, -p-toluidine oder -3,5-xylidine, p-(N,N-dialkylamino-phenylethanol, -benzoesäurederivate, -benzaldehyde, -phenylessigsäureester oder -phenylpropionsäureester. Konkrete Beispiele hierfür sind N,N-Dimethylanilin, N,N-Dimethyl-p-toluidin, N,N-3,5-Tetramethylanilin, N,N-Dimethylamino-p-benzaldehyd, p-(Dimethylamino)benzoesäureethylester oder p-(Dimethylamino)benzonitril. Geeignet sind auch tertiäre aliphatische Amine, wie z.B. Tri-n-butylamin, Dimethylamino-ethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, oder Aminosäure-Derivate, wie z.B. N-Phenylglycin. Vorzugsweise ist der Aminbeschleuniger ausgewählt aus der Gruppe bestehend aus p-(Dimethylamino)-benzoesäureethylester, Dimethylaminoethylmethacrylat, N,N-Dimethylanilin, N,N-Dimethyl-p-toluidin, Triethanolamin und Mischungen davon.
Zusammensetzungen, die saure Monomere als Bindemittel enthalten, wie z.B. selbsthaftende Komposite, können zusätzlich zu oder anstelle von Aminbeschleunigern auch aminfreie Beschleuniger enthalten, wie z.B. Sulfinsäuren bzw. Sulfinate, Borate, Enolate, Phosphine oder andere Verbindungen, die aktive Wasserstoffatome enthalten, wie heterocylische Verbindungen, z.B. Morpholin-Derivate oder 1,3-Dioxolane.
Geeignete Acyl- oder Bisacylphosphinoxide sind u.a. in der EP 0 007 508, EP 0 073 413 A2 (S. 2, Z. 27 bis S. 3, Z. 14; S. 3, Z. 1-13), EP 0 184 095 A2 (S. 2, Z. 4 bis S. 3, Z. 19; S. 4, Z. 34 bis S. 5, Z. 26) und EP 0 615 980 A2 (insbesondere S. 2, Z. 21-35; S. 3, Z. 57 bis S. 5, Z. 28) aufgeführt. Konkrete Beispiele sind die kommerziell erhältlichen Verbindungen 2,4,6-Trimethylbenzoyldiphenylphosphinoxid (Lucirin® TPO, BASF) und Bis(2,4,6-trimethylbenzoyl)phenylphosphinoxid (Irgacure® 819, Ciba). Somit kann die Photoinitiatormischung der erfindungsgemäßen Zusammensetzung gemäß einer Ausführungsform ein Acyl- oder Bisacylphosphinoxid ausgewählt aus der Gruppe bestehend aus 2,4,6-Trimethylbenzoyldiphenylphosphinoxid, Bis(2,4,6-trimethylbenzoyl)phenylphosphinoxid und Mischungen davon enthalten. Vorzugsweise enthält die Photoinitiatormischung ein Bisacylphosphinoxid und besonders bevorzugt Bis(2,4,6-trimethylbenzoyl)phenylphosphinoxid.
Die erfindungsgemäßen Zusammensetzungen enthalten neben der Photoinitiatormischung ein polymerisierbares Bindemittel. Bevorzugt sind Bindemittel auf Basis radikalisch polymerisierbarer Monomere und/oder Prepolymere.

Als radikalisch polymerisierbare Bindemittel eignen sich besonders mono- oder multifunktionelle (Meth)acrylate oder deren Mischungen. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden. Geeignete Beispiele sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat SR-348c (Sartomer) mit 3 Ethoxygruppen oder 2,2-Bis[4-(2-methacryloxypropoxy)phenyl]propan, UDMA (ein Additionsprodukt aus 2-Hydroxyethylmethacrylat und 2,2,4-Trimethylhexamethylendiisocyanat), Di-, Tri- oder Tetraethylenglycol-di(meth)acrylat, Trimethylolpropantri(meth)acrylat, Penta-erythrittetra(meth)acrylat, sowie Glycerindi- und trimethacrylat, 1,4-Butandioldi(meth)acrylat, 1,10-Decandiol-di(meth)acrylat (D₃MA) oder 1,12-Dodecandioldi(meth)acrylat. Bevorzugte (Meth)acrylatmonomere sind Benzyl-, Tetrahydrofurfuryl- oder Isobornylmethacrylat, p-Cumyl-phenoxyethylenglycolmethacrylat, 2,2-Bis[4-(2-methacryloxy-propoxy)phenyl]propan, Bis-GMA, UDMA, SR-348c und D₃MA.

Als radikalisch polymerisierbares Bindemittel lassen sich auch N-mono- oder N-disubstitiuierte Acrylamide, wie z.B. N-Ethylacrylamid oder N,N-Dimethacrylamid, oder Bisacrylamide, wie z.B. N,N'-Diethyl-1,3-bis(acrylamido)propan, 1,3-Bis(methacrylamido)propan, 1,4-Bis(acrylamido)butan oder 1,4-Bis(acryloyl)piperazin einsetzen.

Weiterhin lassen sich als radikalisch polymerisierbares Bindemittel auch bekannte schrumpfungsarme radikalisch ringöffnend polymerisierbare Monomere, wie z.B. mono- oder multifunktionelle Vinylcyclopropane bzw. bicyclische Cyclopropanderivate (vgl. DE 196 16 183 C2 bzw. EP 03 022 855), oder cyclische Allylsulfide (vgl. US 6,043,361 oder US 6,344,556) einsetzen, die darüber hinaus auch in Kombination mit den voranstehend aufgeführten Di(meth)acrylat-Vernetzern verwendet werden können.

Außerdem können als radikalisch polymerisierbares Bindemittel auch radikalisch polymerisierbare Polysiloxane, die aus geeigneten Methacrylsilanen, wie z.B. 3-(Methacryloyloxy)-propyltrimethoxysilan, hergestellt werden können und z.B. in der DE 199 03 177 C2 beschrieben sind, eingesetzt werden.

Für selbsthaftende Komposite lassen sich als radikalisch polymerisierbares Bindemittel auch Mischungen der voranstehend genannten Monomeren mit weiteren radikalisch polymerisierbaren, säuregruppenhaltigen Haftmonomeren verwenden. Geeignete säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin bzw. 4-Vinylbenzoesäure. Beispiele für geeignete Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- oder -2,4,6-trimethylphenylester. Beispiele für geeignete acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Beispiele für geeignete polymerisationsfähige Sulfonsäuren sind Vinylsulfonsäure, 4-Vinylphenylsulfonsäure oder 3-(Methacrylamido)propylsulfonsäure.

Erfindungsgemäß sind Zusammensetzungen bevorzugt, die einen oder mehrere Füllstoffe, vorzugsweise organische oder anorganische partikuläre Füllstoffe enthalten. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure, sowie Minifüllstoffe, wie Quarz-, Glaskeramik- oder Glaspulver mit einer durchschnittlichen Teilchengrösse von 0,01 bis 15 µm, sowie röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid.

Insbesondere wenn die erfindungsgemäße Zusammensetzung zur Anwendung als dentaler Zement oder dentaler Komposit vorgesehen wird, enthält sie vorzugsweise 30 bis 90 Gew.-%, besonders bevorzugt 60 bis 90 Gew.-% mindestens eines Füllstoffs, bezogen auf die Gesamtmasse der Zusammensetzung.

Außerdem können die erfindungsgemäßen Zusammensetzungen im Bedarfsfalle weitere Additive enthalten. Die Additive sind vorzugsweise aus Stabilisatoren, Farbmitteln, mikrobioziden Wirkstoffen, fluoridionenabgebenden Additiven, optischen Aufhellern, Weichmachern, UV-Absorbern und Mischungen davon ausgewählt.

Die erfindungsgemäßen Zusammensetzungen eignen sich besonders als Dentalwerkstoffe, Adhäsive, Beschichtungen, Zemente, Komposite oder Formteile, insbesondere als Zemente und Komposite und ganz besonders als Füllungskomposite.

Zusammensetzungen zur Anwendung als Zemente oder Komposite enthalten vorzugsweise
0,002 bis 5 Gew.-% Photoinitiatormischung,
9 bis 59,8 Gew.-% polymerisierbares Bindemittel und
30 bis 90 Gew.-% Füllstoff enthält, jeweils bezogen auf die Gesamtmasse der Zusammensetzung.

In einer weiteren bevorzugten Ausführungsform enthalten Zusammensetzungen zur Anwendung als Zemente oder Komposite
0,002 bis 5 Gew.-% Photoinitiatormischung,
9 bis 39,8 Gew.-% polymerisierbares Bindemittel und
60 bis 90 Gew.-% Füllstoff enthält, jeweils bezogen auf die Gesamtmasse der Zusammensetzung.

Somit sind Zusammensetzungen besonders bevorzugt, die
(a) 0,2 bis 0,5 Gew.-% (bezogen auf die Masse des polymerisierbaren Bindemittels) Diacylgermanium-Verbindung der Formel (I), vorzugsweise Diacylgermanium-Verbindung der Formel (II), und besonders bevorzugt Diacylgermanium-Verbindung ausgewählt aus der Gruppe bestehend aus Bis(4-methoxybenzoyl)diethylgermanium, Dibenzoyldiethylgermanium und Mischungen davon,
(b) 0,2 bis 0,5 Gew.-% (bezogen auf die Masse des polymerisierbaren Bindemittels) α-Diketon,
(c) 0,4 bis 0,8 Gew.-% (bezogen auf die Masse des polymerisierbaren Bindemittels) Aminbeschleuniger,
(d) 0,1 bis 0,6 Gew.-% (bezogen auf die Masse des polymerisierbaren Bindemittels) Acyl- oder Bisacylphosphinoxid,
(e) 9 bis 59,8 Gew.-% (bezogen auf die Gesamtmasse der Zusammensetzung) polymerisierbares Bindemittel und
(f) 37,8 bis 90 Gew.-% (bezogen auf die Gesamtmasse der Zusammensetzung) Füllstoff enthält.

Die Bestandteile (a) bis (f) dieser Ausführungsform können selbstverständlich unabhängig voneinander eine der oben genannten bevorzugten Bedeutungen haben. Mithin enthält die erfindungsgemäße Zusammensetzung vorzugsweise
(a) 0,2 bis 0,5 Gew.-% (bezogen auf die Masse des polymerisierbaren Bindemittels) Diacylgermanium-Verbindung der Formel (II), vorzugsweise Diacylgermanium-Verbindung ausgewählt aus der Gruppe bestehend aus Bis(4-methoxybenzoyl)diethylgermanium, Dibenzoyldiethylgermanium und Mischungen davon, besonders bevorzugt Bis(4-methoxybenzoyl)diethylgermanium,
(b) 0,2 bis 0,5 Gew.-% (bezogen auf die Masse des polymerisierbaren Bindemittels) α-Diketon ausgewählt aus der Gruppe bestehend aus Campherchinon, 1-Phenylpropan-1,2-dion und Mischungen davon, vorzugsweise Campherchinon,
(c) 0,4 bis 0,8 Gew.-% (bezogen auf die Masse des polymerisierbaren Bindemittels) Aminbeschleuniger ausgewählt aus der Gruppe bestehend aus p-(Dimethylamino)-benzoesäureethylester, Dimethylaminoethylmethacrylat, N,N-Dimethylanilin, N,N-Dimethyl-p-toluidin, Triethanolamin und Mischungen davon, vorzugsweise p-(Dimethylamino)-benzoesäureethylester,
(d) 0,1 bis 0,6 Gew.-% (bezogen auf die Masse des polymerisierbaren Bindemittels) Acyl- oder Bisacylphosphinoxid ausgewählt aus der Gruppe bestehend aus 2,4,6-Trimethylbenzoyldiphenylphosphinoxid, Bis(2,4,6-trimethylbenzoyl)phenylphosphinoxid und Mischungen davon, vorzugsweise Bis(2,4,6-trimethylbenzoyl)phenylphosphinoxid,
(e) 9 bis 59,8 Gew.-% (bezogen auf die Gesamtmasse der Zusammensetzung) polymerisierbares Bindemittel und
(f) 37,8 bis 90 Gew.-% (bezogen auf die Gesamtmasse der Zusammensetzung) Füllstoff.

In einer weiteren bevorzugten Ausführungsform enthält die Zusammensetzung somit
(a) 0,1 bis 0,5 Gew.-% (bezogen auf die Masse des polymerisierbaren Bindemittels) Diacylgermanium-Verbindung der Formel (II), vorzugsweise Diacylgermanium-Verbindung ausgewählt aus der Gruppe bestehend aus Bis(4-methoxybenzoyl)diethylgermanium, Dibenzoyldiethylgermanium und Mischungen davon, besonders bevorzugt Bis(4-methoxybenzoyl)diethylgermanium,
(b) 0,2 bis 0,6 Gew.-% (bezogen auf die Masse des polymerisierbaren Bindemittels) α-Diketon ausgewählt aus der Gruppe bestehend aus Campherchinon, 1-Phenylpropan-1,2-dion und Mischungen davon, vorzugsweise Campherchinon,
(c) 0,3 bis 1,5 Gew.-% (bezogen auf die Masse des polymerisierbaren Bindemittels) Aminbeschleuniger ausgewählt aus der Gruppe bestehend aus p-(Dimethylamino)-benzoesäureethylester, Dimethylaminoethylmethacrylat, N,N-Dimethylanilin, N,N-Dimethyl-p-toluidin, Triethanolamin und Mischungen davon, vorzugsweise p-(Dimethylamino)-benzoesäureethylester,
(d) 0 bis 1,5 Gew.-% (bezogen auf die Masse des polymerisierbaren Bindemittels) Acyl- oder Bisacylphosphinoxid ausgewählt aus der Gruppe bestehend aus 2,4,6-Trimethylbenzoyldiphenylphosphinoxid, Bis(2,4,6-trimethylbenzoyl)phenylphosphinoxid und Mischungen davon, vorzugsweise Bis(2,4,6-trimethylbenzoyl)phenylphosphinoxid,
(e) 9 bis 39,8 Gew.-% (bezogen auf die Gesamtmasse der Zusammensetzung) polymerisierbares Bindemittel und
(f) 60 bis 90 Gew.-% (bezogen auf die Gesamtmasse der Zusammensetzung) Füllstoff.

In einer weiteren besonders bevorzugten Ausführungsform enthält die Zusammensetzung somit
(a) 0,2 bis 0,5 Gew.-% (bezogen auf die Masse des polymerisierbaren Bindemittels) Diacylgermanium-Verbindung der Formel (II), vorzugsweise Diacylgermanium-Verbindung ausgewählt aus der Gruppe bestehend aus Bis(4-methoxybenzoyl)diethylgermanium, Dibenzoyldiethylgermanium und Mischungen davon, besonders bevorzugt Bis(4-methoxybenzoyl)diethylgermanium,
(b) 0,2 bis 0,5 Gew.-% (bezogen auf die Masse des polymerisierbaren Bindemittels) α-Diketon ausgewählt aus der Gruppe bestehend aus Campherchinon, 1-Phenylpropan-1,2-dion und Mischungen davon, vorzugsweise Campherchinon,
(c) 0,4 bis 0,8 Gew.-% (bezogen auf die Masse des polymerisierbaren Bindemittels) Aminbeschleuniger ausgewählt aus der Gruppe bestehend aus p-(Dimethylamino)-benzoesäureethylester, Dimethylaminoethylmethacrylat, N,N-Dimethylanilin, N,N-Dimethyl-p-toluidin, Triethanolamin und Mischungen davon, vorzugsweise p-(Dimethylamino)-benzoesäureethylester,
(d) 0,1 bis 0,6 Gew.-% (bezogen auf die Masse des polymerisierbaren Bindemittels) Acyl- oder Bisacylphosphinoxid ausgewählt aus der Gruppe bestehend aus 2,4,6-Trimethylbenzoyldiphenylphosphinoxid, Bis(2,4,6-trimethylbenzoyl)phenylphosphinoxid und Mischungen davon, vorzugsweise Bis(2,4,6-trimethylbenzoyl)phenylphosphinoxid,
(e) 9 bis 39,8 Gew.-% (bezogen auf die Gesamtmasse der Zusammensetzung) polymerisierbares Bindemittel und
(f) 60 bis 90 Gew.-% (bezogen auf die Gesamtmasse der Zusammensetzung) Füllstoff.

Gegenstand der Erfindung ist auch ein Verfahren zur Herstellung eines Komposits, bei dem eine erfindungsgemäße Zusammensetzung bereitgestellt und anschließend ganz oder teilweise gehärtet wird.

Zur Initiierung der Härtung durch radikalische Polymerisation wird die Photoinitiatormischung der Zusammensetzung vorzugsweise mit Licht im Wellenlängenbereich von 200 bis 750 nm, besonders bevorzugt 200 bis 550 nm, weiter bevorzugt 300 bis 550 nm und ganz besonders bevorzugt 350 bis 500 nm bestrahlt. Besonders vorteilhaft wird dazu eine LED-Lichtquelle mit einer Wellenlänge im Bereich von 380 nm bis 750 nm, vorzugsweise etwa 380 nm bis etwa 490 nm verwendet. Die Aushärtungszeit beträgt vorzugsweise etwa 1 bis 20s.

Die Erfindung wird im Folgenden anhand von Beispielen näher erläutert.

### Beispiele

### Beispiel 1: Lichthärtende Komposite auf Basis einer ternären Initiatorzusammensetzung

Mittels eines Kneters (Firma Linden) wurden vier Komposite auf der Basis von, bezogen auf die Gesamtmasse des Komposits, 70 Gew.-% eines silanisierten Ba-Al-Borosilikatglasfüllers (Schott) mit einer mittleren Partikelgrösse von 1.0 µm und 30 Gew.-% einer Mischung aus Methacrylatmonomeren und Photoinitiator hergestellt. Die Methacrylatmonomermischung enthielt, bezogen auf ihr Gesamtgewicht, 4 Gew.-% Bis-GMA (Esschem), 75 Gew.-% UDMA (Ivoclar Vivadent AG) und 21 Gew.-% Triethylenglycoldimethacrylat (Esschem). Als Photoinitiator wurde der voranstehenden Methacrylatmischung entsprechend der nachfolgend aufgeführten Tabelle 1 eine Mischung aus Campherchinon (CQ) und dem Amin (4-Dimethylamino)-benzoesäureethylester (EMBO) (Komposit A), der Germanium-Photoinitiator Bis-(4-methoxybenzoyl)diethylgermanium (BMBDGe, Ivoclar Vivadent AG) (Komposite B und C) oder einer Mischung aus BMBDGe, CQ und EMBO (Komposit D) verwendet. Die in Tabelle 1 angegebenen Mengen der Initiatorkomponenten beziehen sich auf das Gesamtgewicht der Mischung aus Methacrylatmonomeren und Initiatorkomponenten, jedoch nicht auf das Gesamtgewicht der Zusammensetzung.

Die Bestimmung der Polymerisationstiefe erfolgte analog der ISO-Norm ISO 4049 - 2009 ("Dentistry - Polymer-based restorative materials"). Dazu wurde in einer Metallform ein zylindrisches Loch (h = 6 mm, d = 4 mm) mit Komposit gefüllt und mit einer transparenten Folie abgedeckt. Gegen einen weißen Untergrund wurde dann das Komposit 10 s mit einer dentalen LED-Lichtquelle (Bluephase 20i, Ivoclar Vivadent AG, 1000 mW/cm², 385 - 515 nm) belichtet. Im Anschluss daran wurde das Komposit aus der Metallform entfernt und der nicht polymerisierte Anteil mit einem Kunststoffspatel vom ausgehärteten Teil abgekratzt. Mit einer Schublehre wird die Dicke des ausgehärteten Prüfkörpers bestimmt und als Polymerisationstiefe in Tabelle 1 angegeben. Zur besseren Darstellung der Unterschiede wurde darauf verzichtet, die Schichtdicke durch 2 zu dividieren.

**Tabelle 1: Initiatorgehalt im Monomer der Komposite und Polymerisationstiefe**

| | **BMBDGe** | **CQ** | **EMBO** | **Polymerisationstiefe** |
|---|---|---|---|---|
| | [Gew.-%] | [Gew.-%] | [Gew.-%] | [mm] |
| A* | - | 0,3 | 0, 6 | 4,7 |
| B* | 0,2 | - | | 4,7 |
| C* | 0,4 | - | | 5,1 |
| D | 0,4 | 0,3 | 0,6 | 5,6 |

| | | | | |
|---|---|---|---|---|
| *Vergleichsbeispiel | | | | |

Die erhaltenen Ergebnisse zeigen eine signifikant erhöhte Polymerisationstiefe der Komposite mit einer erfindungsgemäßen ternären Photoinitiator-Zusammensetzung im Vergleich zu Kompositen auf der Basis des reinen Campherchinon-Amin-Systems oder des Ge-Photoinitiators BMBDGe.

### Beispiel 2: Lichthärtende Komposite auf der Basis eines Initiators aus 4 Komponenten

Mittels eines Kneters (Firma Linden) wurden zwei Komposite auf der Basis von, bezogen auf die Gesamtmasse des jeweiligen Komposits, 87 Gew.-% einer Füllstoffmischung, nämlich 35 Gew.-% eines Tetric EvoCeram Isofüllers (Ivoclar Vivadent AG), 39 Gew.-% eines silanisierten Ba-Al-Borosilikatglasfüllers (Schott) mit einer mittleren Partikelgrösse von 1.5 µm, 5 Gew.-% Sphärosil (silanisiertes SiO₂-ZrO₂-Mischoxid mit mittlerer Partikelgrösse von 1.2 µm, Tokoyma Soda) und 8 Gew.-% YbF₃ (Ytterbiumtrifluorid mit mittlerer Partikelgrösse von 0.2 µm, Auer Remy), und 13 Gew.-% einer Mischung aus Methacrylatmonomeren und Photoinitator hergestellt. Die Mischung aus Methacrylatmonomeren und Photoinitiator enthielt, bezogen auf ihr Gesamtgewicht, 20 Gew.-% Bis-GMA (Esschem), 39,1 Gew.-% UDMA (Ivoclar Vivadent AG), 20 Gew.-% 2,2-Bis[4-(2-methacryloxypropoxy)phenyl]propan (Ivoclar Vivadent AG) und 20 Gew.-% p-Cumyl-phenoxyethylenglycolmethacrylat (CMP-1E, Kowa Chemical Japan) (Komposit A). Während entsprechend der nachfolgenden Tabelle 2 bei Komposit A als Photoinitiator eine Mischung aus 0,3 Gew.-% Campherchinon (CQ) und 0,6 Gew.-% (4-Dimethylamino)benzoesäureethylester (EMBO) verwendet wurde, enthielt Komposit B auf Kosten von UDMA zusätzlich noch 0,4 Gew.-% des Germanium-Photoinitiators BMBDGe (Bis-(4-methoxybenzoyl)diethylgermanium) und 0,2 Gew.-% des Bisacylphosphinoxids Irgacure 819 (Bis(2,4,6-trimethylbenzoyl)phenylphosphinoxid, Ciba) (Mengenangaben jeweils bezogen auf die Gesamtmasse der Mischung aus Methacrylatmonomeren und Photoinitiator). Die Bestimmung der Polymerisationstiefe erfolgte wie in Beispiel 1 beschrieben.

**Tabelle 2: Initiatorgehalt im Monomer der Komposite und Polymerisationstiefe**

| | **BMBDGe** | **Irgacure 819** | **CQ** | **EMBO** | **Polymerisations tiefe** |
|---|---|---|---|---|---|
| | [Gew.-%] | [Gew.-%] | [Gew.-%] | [Gew.-%] | [mm] |
| A* | - | - | 0,3 | 0,6 | 5,6 |
| B | 0,4 | 0,2 | 0,3 | 0,6 | 7,2 |

| | | | | | |
|---|---|---|---|---|---|
| *Vergleichsbeispiel | | | | | |

Die Ergebnisse zeigen eine signifikante Verbesserung der Polymerisationstiefe im Falle eines erfindungsgemäßen Komposits.

Darüber hinaus wurde gemäß F. Rueggeberg (Dent. Mater. 10 (1994) 282-286) mittels IR-Spektroskopie der Doppelbindungsumsatz in Abhängigkeit von der Schichtdicke gemessen und in Tabelle 3 wiedergegeben. Auch diese Ergebnisse zeigen eine deutlich bessere Aushärtung des erfindungsgemäßen Komposits B.

**Tabelle 3: Doppelbindungsumsatz (Angaben in Mol-%) in Abhängigkeit vom Initiatorsystem und der Schichtdicke der Komposite**

| **Komposit/Schichtdicke** | **1 mm** | **3 mm** | **5 mm** |
|---|---|---|---|
| **A*** (CQ/EMBO) | 55,1 | 52,1 | 47,0 |
| **B** (BMBDGe/Irg 819/CQ/EMBO) | 67,4 | 63,2 | 50,7 |

| | | | |
|---|---|---|---|
| *Vergleichsbeispiel | | | |

Außerdem wurde das Vickershärteprofil der Prüfkörper mit einer Schichtdicke von 4 mm gemäß D.C. Watts et al. (Brit. Dent. J., 1984, 209-215) bestimmt und in Tabelle 4 dargestellt. Auch diese Ergebnisse zeigen eine bessere Aushärtung des Komposits B in der Tiefe:

**Tabelle 4: Vickershärte (VH, angegeben in N/mm²) und Oberflächenhärte von Kompositprüfkörpern (Aushärtung 5 s mit Bluephase 20i)**

| **Komposit** (Initiatorkomponenten) | **VH oben** | **VH unten** | **Oberflächenhärte (%)** |
|---|---|---|---|
| **A*** (CQ/EMBO) | 461 | 370 | 80.3 |
| **B** (BMBDGe/Irg 819/CQ/EMBO) | 552 | 517 | 93.7 |

| | | | |
|---|---|---|---|
| *Vergleichsbeispiel | | | |

Schließlich wurde die Farbänderung der Komposite A und B bei der Härtung untersucht, und die entsprechenden Ergebnisse sind in Tabelle 5 dargestellt.

**Tabelle 5: Farb-Werte der Komposite A und B vor und nach der Aushärtung (5 s mit Bluephase 20i)**

| | **a-Wert** | **b-Wert** |
|---|---|---|
| Kompositpaste | | |
| Komposit **A*** | -2,76 | 22,34 |
| Komposit **B** | -5,18 | 29,95 |
| Kompositprüfkörper | | |
| Komposit **A*** | -0,72 | 11,33 |
| Komposit **B** | -0,75 | 12,38 |

| | | |
|---|---|---|
| *Vergleichsbeispiel | | |

Die Paste des Komposits B mit der erfindungsgemäßen Initiatorzusammensetzung hat im Vergleich zu Komposit A deutlich höhere a- und b-Werte. Aufgrund des guten Bleichverhaltens der erfindungsgemäßen Zusammensetzung sind die a- und b-Werte im ausgehärteten Prüfkörper jedoch wesentlich kleiner und es bestehen kaum noch Unterschiede zum Komposit A.

### Beispiel 3: Aushärtung eines Komposites mit Grünlicht (λₘₐₓ = 517 nm) und anschliessender Nachhärtung mit Blaulicht (λₘₐₓ = 430 nm)

Entsprechend Beispiel 2 wurden zwei Komposite gleicher Monomer- und Füllstoffzusammensetzung hergestellt, wobei beim Komposit A (Vergleichsbeispiel) als Photioinitiator eine Mischung aus 0,3 Gew.-% CQ und 0,6 Gew.-% EMBO verwendet wurde und im Komposit C eine Mischung aus 0,3 Gew.-% Campherchinon (CQ), 0,6 Gew.-% (4-Dimethylamino)benzoesäureethylester (EMBO) und 0,4 Gew.-% des Germanium-Photoinitiators BMBDGe (Bis-(4-methoxybenzoyl)diethylgermanium) verwendet wurde. Bei einer ersten Versuchsserie wurden die Komposite zunächst mit einer grünen LED (λₘₐₓ = 517 nm) 2x 20 s belichtet und dann die Biegfestigkeit und der Biege-E-Modul bestimmt. In einer zweiten Versuchsserie erfolgte nach der Belichtung mit der grünen LED (2x20 s) eine gleich lange Belichtung mit einer blauen LED (λₘₐₓ = 430 nm). Die Ergebnisse der mechanischen Untersuchungen sind in Tabelle 6 dargestellt.

**Tabelle 6: Mechanische Eigenschaften der Komposite A und C nach ein- und zweistufiger Aushärtung**

| Eigenschaften/Komposit | Aushärtung mit grüner LED (λₘₐₓ = 517 nm) | Aushärtung mit blauer LED (λₘₐₓ = 430 nm) |
|---|---|---|
| Biegefestigkeit (MPa) | | |
| Komposit A* | 40.8 ± 10.8 | 114.5 ± 5.3 |
| Komposit C | 49.4 ± 6.4 | 121.0 ± 3.8 |
| Biege-E-Modul (MPa) | | |
| Komposit A* | 1750 ± 420 | 6930 ± 310 |
| Komposit C | 2250 ± 390 | 9590 ± 410 |

| | | |
|---|---|---|
| *Vergleichsbeispiel | | |

Die Ergebnisse zeigen, dass mit einem Photoinitiator, der neben CQ und EMBO noch einen Germanium-Photoinitiator gemäß Formel (I) enthält, durch den Einsatz von zwei Bestrahlungsquellen unterschiedlicher Wellenlängenbereiche eine deutlich bessere zweistufige Aushärtung realisiert werden kann.

## Patentansprüche

1. Zusammensetzung mit mindestens einem polymerisierbaren Bindemittel, **dadurch gekennzeichnet, dass** sie eine Photoinitiatormischung enthält, die bezogen auf das polymerisierbare Bindemittel
(a) 0,2 bis 0,5 Gew.-% mindestens einer Diacylgermanium-Verbindung der Formel (I),
(b) 0,2 bis 0,5 Gew.-% mindestens eines α-Diketons,
(c) 0,4 bis 0,8 Gew.-% mindestens eines Beschleunigers, insbesondere Aminbeschleunigers, und
(d) 0,1 bis 0,6 Gew.-% mindestens eines Acyl- oder Bisacylphosphinoxids
enthält, 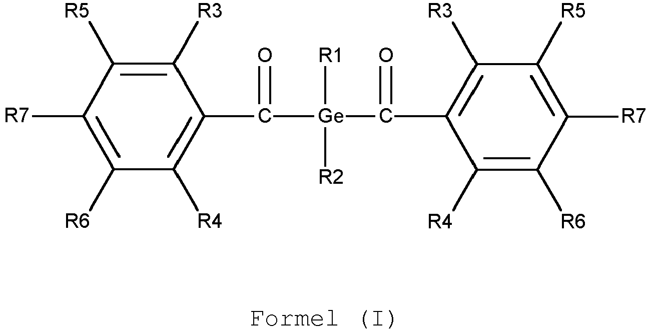 in der
R¹, R² unabhängig voneinander ein linearer C₁₋₄-Alkyl-, oder C₂₋₄-Alkenyl-Rest sind, der durch eine oder mehrere polymerisationsfähige Gruppen substituiert sein kann;
R³, R⁴ unabhängig voneinander jeweils H, Halogen, ein verzweigter oder linearer C₁₋₄-Alkyl- oder -O-C₁₋₄-Alkylrest sind;
R⁵, R⁶ unabhängig voneinander jeweils H, Halogen, ein linearer C₁₋₂₀-Alkyl-, -Alkenyl-, -Alkoxy- oder -Alkenoxy-Rest sind, der durch ein oder mehrere -O-, -S- oder -NR⁸-Reste unterbrochen und durch ein oder mehrere polymerisationsfähige Gruppen substituiert sein kann;
R⁷ unabhängig voneinander jeweils H, Halogen, ein linearer C₁₋₂₀-Alkyl-, -Alkenyl-, -Alkoxy- oder -Alkenoxy-Rest ist, der durch ein oder mehrere -O-, -S- oder -NR⁸-Reste unterbrochen und durch ein oder mehrere polymerisationsfähige Gruppen substituiert sein kann; und
R⁸ unabhängig jeweils H, ein C₁₋₂₀-Alkyl-, -Alkenyl-, -Alkoxy- oder -Alkenoxy-Rest ist.

2. Zusammensetzung nach Anspruch 1, bei der die Diacylgermanium-Verbindung eine Verbindung der Formel (II) ist 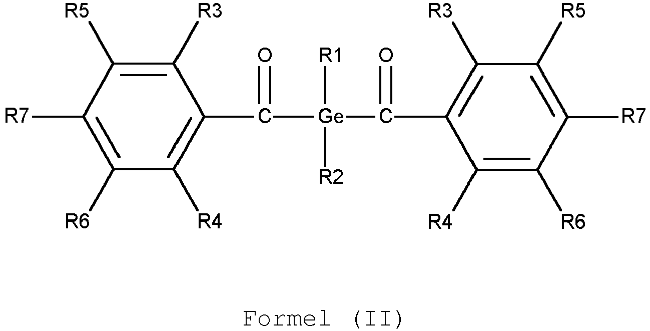 in der
R¹, R² unabhängig voneinander ein linearer C₁₋₄-Alkyl-Rest sind;
R³, R⁴ jeweils H sind;
R⁵, R⁶ jeweils H sind; und
R⁷ unabhängig voneinander jeweils H, ein linearer C₁₋₆-Alkyl- oder -Alkoxy-Rest ist.

3. Zusammensetzung nach Anspruch 2, bei die Diacylgermanium-Verbindung ausgewählt ist aus der Gruppe bestehend aus Bis(4-methoxybenzoyl)diethylgermanium, Dibenzoyldiethylgermanium und Mischungen davon.

4. Zusammensetzung nach einem der Ansprüche 1 bis 3, bei der das α-Diketon ausgewählt ist aus der Gruppe bestehend aus Campherchinon, 1-Phenylpropan-1,2-dion und Mischungen davon.

5. Zusammensetzung nach einem der Ansprüche 1 bis 4, bei der der Beschleuniger ein Aminbeschleuniger ist und der Aminbeschleuniger vorzugsweise ausgewählt ist aus der Gruppe bestehend aus p-(Dimethylamino)-benzoesäureethylester, Dimethylaminoethylmethacrylat, N,N-Dimethylanilin, N,N-Dimethyl-p-toluidin, Triethanolamin und Mischungen davon.

6. Zusammensetzung nach einem der Ansprüche 1 bis 5, bei der das Acyl- oder Bisacylphosphinoxid ausgewählt ist aus der Gruppe bestehend aus 2,4,6-Trimethylbenzoyldiphenylphosphinoxid, Bis(2,4,6-trimethylbenzoyl)phenylphosphinoxid und Mischungen davon.

7. Zusammensetzung nach einem der Ansprüche 1 bis 6, die als polymerisierbares Bindemittel mindestens ein radikalisch polymerisierbares Monomer und/oder Prepolymer enthält.

8. Zusammensetzung nach Anspruch 7, die als Bindemittel ein mono- oder multifunktionelles (Meth)acrylat, ein N-mono- oder -disubstituiertes Acrylamid, ein Bisacrylamid, ein radikalisch ringöffnend polymersierbares Monomer, ein cyclisches Allylsulfid, ein Polysiloxan auf Basis von Methacrylsilanen oder eine Mischung davon oder eine Mischung mindestens eines der Bindemittel mit einem radikalisch polymerisierbaren, säuregruppehaltigen Monomeren enthält.

9. Zusammensetzung nach Anspruch 7 oder 8, die als Bindemittel ein mono- oder multifunktionelles (Meth)acrylat oder eine Mischung davon enthält.

10. Zusammensetzung nach einem der Ansprüche 1 bis 9, die zusätzlich Füllstoff enthält.

11. Zusammensetzung nach einem der Ansprüche 1 bis 10, die zusätzlich mindestens ein Additiv enthält, das aus Stabilisatoren, Farbmitteln, mikrobioziden Wirkstoffen, fluoridionenabgebenden Additiven, optischen Aufhellern, Weichmachern, UV-Absorbern und Mischungen davon ausgewählt ist.

12. Zusammensetzung nach einem der Ansprüche 10 oder 11, die 0,002 bis 5 Gew.-% Photoinitiatormischung,
9 bis 59,8 Gew.-% polymerisierbares Bindemittel und 30 bis 90 Gew.-% Füllstoff enthält, jeweils bezogen auf die Gesamtmasse der Zusammensetzung.

13. Verwendung einer Zusammensetzung gemäß einem der Ansprüche 1 bis 12 zur Herstellung von Adhäsiven, Beschichtungen, Zementen, Kompositen, Formteilen oder Dentalwerkstoffen.

14. Verfahren zur Herstellung eines Komposits, bei dem man eine Zusammensetzung gemäß einem der Ansprüche 1 bis 12 bereitstellt und anschließend ganz oder teilweise härtet.

## Claims

1. Composition with at least one polymerisable binder, **characterized in that** said composition comprises a photoinitiator mixture that, relative to the polymerisable binder, comprises
(a) 0.2 to 0.5 wt % of at least one diacylgermanium compound of Formula (I),
(b) 0.2 to 0.5 wt % of at least one α-diketone,
(c) 0.4 to 0.8 wt % of at least one accelerator, in particular an amine accelerator and
(d) 0.1 to 0.6 wt % of at least one acyl- or bisacylphosphine oxide, 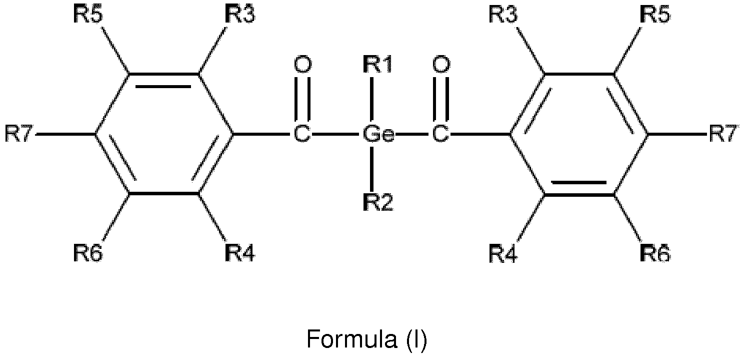
in which
R¹, R² independently of each other are a linear C₁₋₄ alkyl, or C₂₋₄ alkenyl group, which may be substituted by one or more polymerisable groups;
R³, R⁴ independently of each other are each H, halogen, a branched or linear C₁₋₄ alkyl or -O-C₁₋₄ alkyl group;
R⁵, R⁶ independently of each other are each H, halogen, a linear C₁₋₂₀ alkyl, -alkenyl, -alkoxy or -alkenoxy group, which may be interrupted by one or more -O-, -S- or -NR⁸- moieties and substituted by one or more polymerisable groups;
R⁷ independently of each other is H, halogen, a linear C₁₋₂₀ alkyl,-alkenyl, -alkoxy or -alkenoxy group, which may be interrupted by one or more -O-, -S- or NR⁸- moieties and substituted by one or more polymerisable groups; and
R⁸ independently is H, a C₁₋₂₀ alkyl, -alkenyl, -alkoxy or -alkenoxy group.

2. Composition according to claim 1 in which the diacylgermanium compound is a compound of Formula (II) 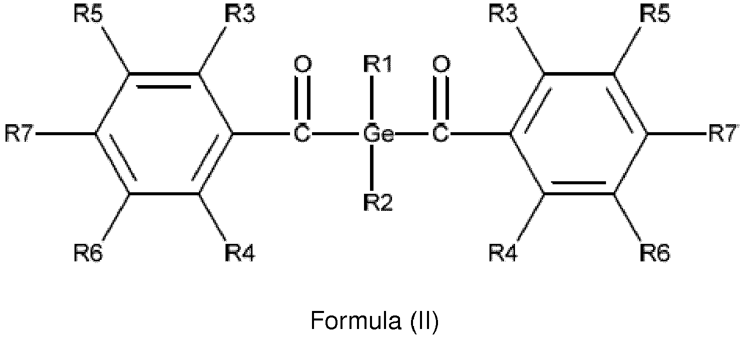 in which
R¹, R² independently of each other are a linear C₁₋₄ alkyl group;
R³, R⁴ are each H;
R⁵, R⁶ are each H; and
R⁷ independently of each other are each H, a linear C₁₋₆ alkyl or-alkoxy group.

3. Composition according to claim 2, in which the diacylgermanium compound is selected from the group consisting of bis(4-methoxybenzoyl)diethylgermanium, dibenzoyldiethylgermanium and mixtures thereof.

4. Composition according to one of claims 1 to 3, in which the α-diketone is selected from the group consisting of camphorquinone, 1-phenylpropane-1,2-dione and mixtures thereof.

5. Composition according to one of claims 1 to 4, in which the accelerator is an amine accelerator and the amine accelerator is preferably selected from the group consisting of *p*-(dimethylamino) benzoic acid ethyl ester, dimethylaminoethyl methacrylate, *N*,*N*-dimethylaniline, *N*,*N*-dimethyl-*p-*toluidine, triethanolamine and mixtures thereof.

6. Composition according to one of claims 1 to 5, in which the acyl- or bisacylphosphine oxide is selected from the group consisting of 2,4,6-trimethylbenzoyl diphenylphosphine oxide, bis(2,4,6-trimethylbenzoyl)phenylphosphine oxide and mixtures thereof.

7. Composition according to one of claims 1 to 6 which comprises at least one radically polymerisable monomer and/or prepolymer as the polymerisable binder.

8. Composition according to claim 7 which comprises as the binder a mono- or multifunctional (meth)acrylate, an *N*-mono- or -disubstituted acrylamide, a bisacrylamide, a radically ring-opening polymerisable monomer, a cyclic allyl sulphide, a polysiloxane based on methacrylic silanes or a mixture thereof or a mixture of at least one of the binders with a radically polymerisable, acid group-containing monomer.

9. Composition according to claim 7 or 8 which comprises a mono- or multifunctional (meth)acrylate or a mixture thereof as the binder.

10. Composition according to one of claims 1 to 9 which additionally comprises filler.

11. Composition according to one of claims 1 to 10 which additionally comprises at least one additive selected from stabilisers, dyes, microbiocidially-active ingredients, fluoride ion-releasing additives, optical brighteners, plasticisers, UV absorbers and mixtures thereof.

12. Composition according to one of claims 10 or 11 which comprises
0.002 to 5 wt % photoinitiator mixture,
9 to 59.8 wt % polymerisable binder and
30 to 90 wt % filler,
in each case relative to the total mass of the composition.

13. Use of a composition according to one of claims 1 to 12 for producing adhesives, coatings, cements, composites, shaped parts or dental materials.

14. Process for producing a composite, in which process the composition according to one of claims 1 to 12 is produced and then completely or partially cured.

## Revendications

1. Composition comportant au moins un liant polymérisable, **caractérisée en ce qu'**elle contient un mélange de photoamorceurs qui contient, par rapport au liant polymérisable,
(a) 0,2 à 0,5 % en poids d'au moins un composé diacylgermanium de formule (I),
(b) 0,2 à 0,5 % en poids d'au moins une α-dicétone,
(c) 0,4 à 0,8 % en poids d'au moins un accélérateur, en particulier un accélérateur de type aminé, et
(d) 0,1 à 0,6 % en poids d'au moins un oxyde d'acylphosphine ou de bisacylphosphine, 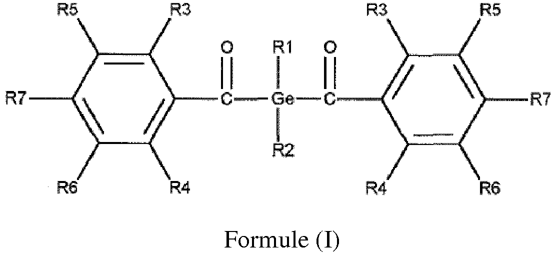
dans laquelle
R¹ , R² représentent indépendamment l'un de l'autre un radical alkyle en C₁-C₄ ou alcényle en C₂-C₄ linéaire, qui peut porter comme substituant(s) un ou plusieurs groupe(s) apte(s) à la polymérisation ;
R³, R⁴ représentent chacun indépendamment l'un de l'autre un atome d'hydrogène ou d'halogène, un radical alkyle en C₁-C₄ ou -O-alkyle en C₁-C₄, linéaire ou ramifié ;
R⁵, R⁶ représentent chacun indépendamment l'un de l'autre un atome d'hydrogène ou d'halogène, un radical alkyle, alcényle, alcoxy ou alcénoxy en C₁-C₂₀ linéaire, qui peut être interrompu par un ou plusieurs atome(s) d'oxygène ou de soufre ou radical/radicaux -NR⁸ et porter comme substituant(s) un ou plusieurs groupe(s) apte(s) à la polymérisation ;
R⁷ représente chaque fois indépendamment un atome d'hydrogène ou d'halogène, un radical alkyle, alcényle, alcoxy ou alcénoxy en C₁-C₂₀ linéaire, qui peut être interrompu par un ou plusieurs atome(s) d'oxygène ou de soufre ou radical/radicaux -NR⁸ et porter comme substituant(s) un ou plusieurs groupe(s) apte(s) à la polymérisation ;
R⁸ représente chaque fois indépendamment un atome d'hydrogène, un radical alkyle, alcényle, alcoxy ou alcénoxy en C₁-C₂₀.

2. Composition selon la revendication 1, dans laquelle le composé diacylgermanium est un composé de formyle (II) 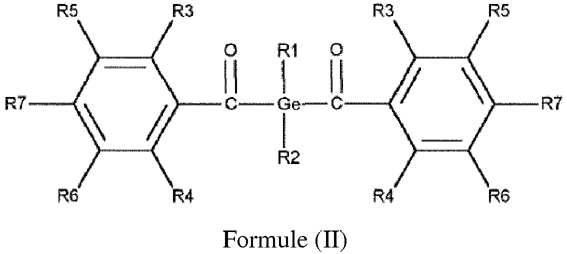 dans laquelle
R¹, R² représentent indépendamment l'un de l'autre un radical alkyle en C₁-C₄ linéaire ;
R³, R⁴ représentent chacun un atome d'hydrogène ;
R, R⁶ représentent chacun un atome d'hydrogène ; et
R⁷ représente chaque fois indépendamment un atome d'hydrogène, un radical alkyle ou alcoxy en C₁-C₆ linéaire.

3. Composition selon la revendication 2, dans laquelle le composé diacylgermanium est choisi dans l'ensemble constitué par le bis(4-méthoxybenzoyl)-diéthylgermanium, le dibenzoyldiéthylgermanium et des mélanges de ceux-ci.

4. Composition selon l'une quelconque des revendications 1 à 3, dans laquelle l'α-dicétone est choisie dans l'ensemble constitué par la camphoquinone, la 1-phénylpropane-1,2-dione et des mélanges de celles-ci.

5. Composition selon l'une quelconque des revendications 1 à 4, dans laquelle l'accélérateur est un accélérateur de type amine et l'accélérateur de type amine est choisi de préférence dans l'ensemble constitué par le p-(diméthylamino)benzoate d'éthyle, le méthacrylate de diméthylaminoéthyle, la N,N-diméthylaniline, la N,N-diméthyl-p-toluidine, la triéthanolamine et des mélanges de ceux-ci.

6. Composition selon l'une quelconque des revendications 1 à 5, dans laquelle l'oxyde d'acylphosphine- ou de bisacylphosphine est choisi dans l'ensemble constitué par l'oxyde de 2,4,6-triméthylbenzoyldiphénylphosphine, l'oxyde de bis(2,4,6-triméthylbenzoyl)phénylphosphine et des mélanges de ceux-ci.

7. Composition selon l'une quelconque des revendications 1 à 6, qui contient en tant que liant polymérisable au moins un prépolymère et/ou un monomère polymérisable par voie radicalaire.

8. Composition selon la revendication 7, qui contient en tant que liant un (méth)acrylate mono- ou multifonctionnel, un acrylamide N-mono- ou N-disubstitué, un bisacrylamide, un monomère polymérisable par voie radicalaire avec ouverture de cycle, un sulfure d'allyle cyclique, un polysiloxane à base de méthacrylsilanes ou un mélange de ceux-ci ou un mélange d'au moins un des liants avec un monomère contenant un groupe acide, polymérisable par voie radicalaire.

9. Composition selon la revendication 7 ou 8, qui contient en tant que liant un (méth)acrylate mono- ou multifonctionnel ou un mélange de tels (méth)acrylates.

10. Composition selon l'une quelconque des revendications 1 à 9, qui contient en outre une charge.

11. Composition selon l'une quelconque des revendications 1 à 10, qui contient en outre au moins un additif, qui est choisi parmi des stabilisants, des agents colorants, des substances actives microbicides, des additifs libérant des ions fluorure, des azurants optiques, des plastifiants, des absorbeurs UV et des mélanges de ceux-ci.

12. Composition selon l'une quelconque des revendications 10 et 11, qui contient
0,002 à 5 % en poids de mélange de photoamorceurs,
9 à 59,8 % en poids de liant polymérisable et
30 à 90 % en poids de charge, chaque fois par rapport à la masse totale de la composition.

13. Utilisation d'une composition selon l'une quelconque des revendications 1 à 12, pour la production d'adhésifs, de revêtements, de ciments, de composites ; de pièces moulées ou de matériaux dentaires.

14. Procédé pour la production d'un composite, dans lequel on prépare durcir une composition selon l'une quelconque des revendications 1 à 12 et ensuite on la fait sécher en partie ou en totalité.
